(19) Europäisches Patentamt
European Patent Office
Office européen des brevets

(11) **EP 3 543 914 A1**

(12) **EUROPEAN PATENT APPLICATION**

(43) Date of publication:
**25.09.2019 Bulletin 2019/39**

(51) Int Cl.:
***G06N 3/00*** *(2006.01)*    ***G06N 5/04*** *(2006.01)*

(21) Application number: **18163225.8**

(22) Date of filing: **22.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **HARMA, Aki Sakari
5656 AE Eindhoven (NL)**
• **HELAOUI, Rim
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **TECHNIQUES FOR IMPROVING TURN-BASED AUTOMATED COUNSELING TO ALTER BEHAVIOR**

(57)    Techniques described herein relate to applying reinforcement learning to improve engagement with counseling chatbots. In various embodiments, based on a first state of a subject and a decision model (109), a given natural language response may be selected (404) from a plurality of candidate natural language responses and provided to the user by the counseling chatbot. A free-form natural language input may be received (408) from the subject at one or more input components of one or more computing devices. A second state of the subject may be determined (410) based on speech recognition output generated from the free-from natural language input. The second state may be a positive, negative, or neutral valance towards a target behavior change. Based on the second state, and instant reward may be calculated (412) and used to train (414) the decision model.

Fig. 4

START

DETERMINE A FIRST STATE OF A USER BASED ON ONE OR MORE SIGNALS
**402**

SELECT, FROM A PLURALITY OF CANDIDATE NATURAL LANGUAGE RESPONSES, BASED ON THE FIRST STATE AND A DECISION MODEL, A GIVEN NATURAL LANGUAGE RESPONSE
**404**

PROVIDE, BY THE COUNSELING CHATBOT, THE GIVEN NATURAL LANGUAGE RESPONSE
**406**

RECEIVE, AT ONE OR MORE INPUT COMPONENTS, A FREE-FORM NATURAL LANGUAGE INPUT FROM THE USER
**408**

DETERMINE A SECOND STATE OF THE USER BASED ON SPEECH RECOGNITION OUTPUT GENERATED FROM THE FREE-FROM NATURAL LANGUAGE INPUT, WHEREIN THE SECOND STATE ALSO COMPRISES A POSITIVE OR NEGATIVE VALANCE TOWARDS A TARGET BEHAVIOR CHANGE
**410**

CALCULATE AN INSTANT REWARD BASED ON THE SECOND STATE
**412**

TRAIN THE DECISION MODEL BASED ON THE INSTANT REWARD
**414**

400

**EP 3 543 914 A1**

**Description**

FIELD OF THE INVENTION

Various embodiments described herein are directed generally to using artificial intelligence to improve turn-based human-to-computer dialogs. More particularly, but not exclusively, various methods and apparatus disclosed herein relate to applying reinforcement learning to improve turn-based counseling with conversational agents.

BACKGROUND OF THE INVENTION

Conventional approaches to help subjects (*e.g.*, patients, clients) change their lifestyle and/or alter their behavior have either been based on health education or health counseling. Passive health education is somewhat effective across a population of subjects, but is less efficient for individual subjects. Health counseling sessions between a human counselor and an individual subject are known to be efficient in creating the required awareness and engagement for a durable change in lifestyle and behaviors. Indeed there is clear evidence that collaborative interview techniques help subjects (*e.g.*, patients, clients) to change health behavior. It is also known that the effectiveness of the counselling increases with hours of exposure, and that there are large individual differences between counselors in their ability to get good results. This may be in part because counseling practice typically lacks formal definitions or theoretical framework. Instead, counselling is an art that is largely defined by a collection of traditions and rituals established by famous practitioners. For example, with motivational interviewing, the goal is to cause the subject to contemplate between positive and negative valence, and ultimately steer them towards "positive valence." Consequently, scaling of human-provided counselling services across large numbers of subjects, especially in a uniform way, is challenging.

Often the practitioners of counseling clinics (i.e., "counselors") describe their techniques as being guided by certain discrete cognitive and emotional states that the subject is assumed to possess in moving towards a target state. In terms of control theory the hypothesized internal states are fundamentally not observable, i.e. they do not exist in any physically measurable sense. Therefore it is difficult to characterize or measure the state of the subject for the design of an automated system.

SUMMARY OF THE INVENTION

The present disclosure is directed to methods and apparatus for applying reinforcement learning to improve turn-based chatbot counseling. For example, in various embodiments, a software application often referred to as a "chatbot" (also referred to as a "virtual assistant," "digital assistant," "digital agent," "conversational agent," etc.) may be configured to act as a "counseling chatbot" that counsels subjects in order to change their behavior, i.e., to achieve a "target behavior change." Counseling chatbots (or "counseling agents") configured with selected aspects of the present disclosure may be configured to counsel subjects to change a variety of different targeted behaviors, many related to health and wellbeing. These targeted behaviors may include but are not limited to smoking, alcohol consumption (or "drinking"), drug use, exercise habits, eating habits, social behaviors, and so forth.

A human-based counseling session-between a human coach and a subject (client)-usually takes place in a temporally-constrained coaching session, *e.g.*, during an appointment having a set length. By contrast, counseling chatbots configured with selected aspects of the present disclosure facilitate a fully automated counseling session between the counseling chatbot (acting as a coach) and the subject (also referred to as the "client," "patient"). One of the technical advantages of this is that an automated counseling session between a counseling chatbot and a subject may have a freer temporal structure than a human-based counseling session because the automated counseling session can continue without limitations of time and/or place. For example, a counseling session started in one place and time can continue in another location, *e.g.,* using a different computing device and/or a different modality *(e.g.,* voice versus typed input). This also gives rise to another technical advantage-allowing contextual focusing of the counseling session. For example, contextual signals other than direct input from the subject, such as sensor signals (*e.g.*, GPS), physiological signals, etc., can be used to determine the subject's state.

In addition, the free temporal structure allows better control of (i.e. reactions to) fluctuations in the behavior of the subject. For example, suppose the subject has a drinking problem. The automated counseling session between the subject and the counseling chatbot may continue over several lapses and "dry" periods of the subject. Techniques described herein enable long-term strategic learning from the ongoing human-to-computer dialog between the subject and the conversational agent, which allows the most efficient personalized support to be provided to the subject. It is also possible to make cumulative progress visible to the subject and/or other relevant stakeholders, such as family, friends, clinicians, counselors, probation officers, etc.

In various embodiments, reinforcement learning may be employed to "train" a counseling chatbot to influence subjects towards target behavior changes. For example, in some embodiments, the counseling chatbot agent may act in accordance with a Markov Decision Process ("MDP"), which is a state model in which a subject (or "client") is considered to be in a particular state $s_k$ at any given turn $k$ of the MDP. At each turn $k$ of the MDP, the subject's observed or inferred state $s_k$ is used, in combination with a decision model, to select a message $y_k$, typically taking the form of natural language output, to be provided to the subject. The decision model may take various forms, such as a decision matrix, a neural network, and so forth.

The message $y_k$ provided to the subject may trigger some sort of action $a_k$ from the subject. An action $a_k$ may be, for instance, a spoken or typed response from the subject made in reaction to the message $y_k$. Each action $a_k$ provided by the subject may be associated with a positive or negative reward, $R(a_k)$, that is collected by the counseling chatbot. In some embodiments, the goal of the counseling chatbot may be to maximize a cumulative mean reward, *e.g.,* given by an equation such as the following:

$$R_c = \frac{1}{K} \sum_{k}^{K} R(a_k)$$

$$(1)$$

More generally, any operator or function $G$ that can be a sum or any other set of arithmetic operations such that $R_c = G(a_0, a_1, a_2, ..., a_{n-1}, a_n)$ can be employed.

In various embodiments, each action $a_k$ by the subject may be classified into one of a plurality of categories, and each of the plurality of categories may be associated with a particular reward value. For example, in some embodiments, each action $a_k$ by the subject may be classified as: a positive valance towards a target behavior change; or a negative valence towards the target behavior change. Additionally or alternatively, in some embodiments, a third category, neutral in relation to the target behavior change, may also be used.

As used herein, a subject demonstrates a "positive valence" towards a target behavior change when the subject takes some action (*e.g.,* a vocal utterance, activity detected by sensors, etc.) that evidences movement towards the target behavior change. For example, a subject that is attempting to quit drinking might say, "rather than having a beer, I'm going to do some yoga." Additionally or alternatively, the subject may not say anything, but one or more sensors associated with one or more computing devices operated by the subject may provide signals that indicate that the subject travelled to, and spent time in, a yoga studio, as opposed to, say, a bar. Either may indicate positive valence towards the target behavior change of quitting drinking.

By contrast, a subject demonstrates a "negative valence" towards a target behavior change when the subject takes some action that evidences movement away from the target behavior change. For example, suppose the same subject who is trying to quit drinking says something like, "Find me directions to the closest liquor store." Or, suppose one or more GPS coordinates detected by the subject's smart phone or watch indicate that the user went to, and spent significant time at, a bar. Either may indicate negative valence towards the target behavior change of quitting drinking. Actions (*e.g.,* subject utterances, activities, etc.) that do not tend to indicate positive or negative valence towards the target behavior change may be considered neutral.

In some embodiments, positive or negative valences may have subclasses which may be subject to different decision models and/or reward functions. For example, a positive valence in relation to a target behavior change such as taking drugs may include the following subclasses: Desire ("I really want to quit"); Ability ("I am able to stop using drugs when I want to"); Reason ("I have to stop doing it because otherwise I lose my license"); Commitment ("I will stop now and never buy drugs again"); and Progress ("I have been drug-free for two weeks"). A negative valence in relation to the same target behavior change may include subclasses such as the following: Enablement ("my friends keep pressuring me to do drugs"); Lack of commitment ("I have no desire to stop"); and so forth. These subclasses may lead to different responses by the counseling chatbot and may have different rewards than the high-level classes.

An action $a_k$ may be classified into a category using various techniques. In some embodiments, natural language processing and/or machine learning techniques may be employed. For example, natural language processing may be employed to annotate textual data (*e.g.,* generated from the subject's vocal utterance) representing a subject's reaction (i.e. action $a_k$) to a particular message $y_k$. Additionally or alternatively, in some embodiments, a machine learning model/classifier such as a neural network may be trained to generate output that is indicative of a category of an action $a_k$ applied across the model as input. For example, supervised training may be employed in which training examples (which may take the form of feature vectors in some embodiments) are labeled with appropriate categories and then applied as input across the model. The output generated based on the model may be compared to the labels (i.e., the appropriate categories) to generate error (or "loss"). This error may then be used to train the model, *e.g.,* by using techniques such as gradient descent *(e.g.,* stochastic or batch) and/or back propagation.

As noted above, the message $y_k$ provided by the counseling chatbot at each turn is governed by a decision model, or more generally, a function, which depends on the state of the subject. The next action $a_k$ (or reaction) from the subject can be then predicted by the model $a_k = F(\bar{s}_k, y_k)$, where $\bar{s}_k$ is an estimate of the state of the subject. As discussed in the background, the internal state of the subject is generally not directly available. Accordingly, in some simplified phenomenological embodiments, the subject's internal state may be associated with the subject's action $a_k$ such that $\bar{s}_k = a_k$. The function $F(a_k, y_k)$ can then be learned from a historical sequence of messages $y$ and reactive actions $a$. In some embodiments, the function $F()$ may then be used for the selection of $y_k$ by the counseling chatbot. In some embodiments, a greedy strategy for the selection of a message $y$ by the counseling chatbot may be based on finding, in each turn, the message y that maximizes $R(F(a_{k-1}, y_k))$. However, various alternative optimization criteria are also contemplated. In some embodiments, the function $F()$ may depend on the history of subject and counseling chatbot utterances $a_{h<k}$ and $y_{h<k}$, measurement data from external sensors, the medical history of the subject, and/or counseling chatbot usage history.

Subjects may interact with counseling chatbots configured with selected aspects of the present disclosure using a variety of device types and/or input/output (I/O) modalities. For example, in some embodiments, a subject may interact with a counseling chatbot vocally using a standalone interactive speaker, an in-vehicle computing device, a home entertainment system, etc. Additionally or alternatively, subjects may interact with the counseling chatbot using other modalities, such as via typed input and visual output.

Generally, in one aspect, a method implemented by one or more processors as part of a human-to-computer dialog between a subject and a counseling chatbot may include: determining a first state of the subject based on one or more signals; selecting, from a plurality of candidate natural language responses, based on the first state and a decision model, a given natural language response; providing, by the counseling chatbot, at one or more output components of one or more computing devices operated by the subject to engage in the human-to-computer dialog with the counseling chatbot, the given natural language response; receiving, at one or more input components of one or more of the computing devices, a free-form natural language input from the subject; determining a second state of the subject based on speech recognition output generated from the free-from natural language input, wherein the second state comprises a positive or negative valance towards a target behavior change; calculating an instant reward based on the second state; and training the decision model based on the instant reward.

In various embodiments, the decision model may include a decision matrix, and training the decision model comprises updating the decision matrix based on the instant reward. In various embodiments, the decision model may include a neural network. In various embodiments, training the neural network may include applying back propagation to adjust one or more weights associated with one or more hidden layers of the neural network, wherein applying the back propagation is based on the instant reward.

In various embodiments, the one or more signals may include speech recognition generated from a first free-form natural language input, and the free-form natural language input comprises a second free-form natural language input. In various embodiments, the method may further include determining, based at least in part on the instant reward and other instance rewards calculated during the human-to-computer dialog, a cumulative reward. In various embodiments, the method may include providing, at one or more visual output components of one or more of the computing devices operated by the subject, a visual indication of the cumulative reward.

In various embodiments, training the decision model may include maximizing a cumulative mean reward $R_C$ given by the following equation (Equation (1) from above):

$$R_c = \frac{1}{K}\sum_{k}^{K} R(a_k)$$

wherein $K$ is a positive integer corresponding to a number of turns in the human-to-computer dialog, and $a_k$ represents an action at a given turn $k$, and $R(a_k)$ represents an instant reward at a given turn $k$.

In various embodiments, the plurality of candidate natural language responses may include: a first set of informational candidate responses; a second set of candidate responses designed to stimulate a response from the subject; and a third set of candidate responses designed to simulate listening or reflection on part of the counseling chatbot.

In addition, some implementations include one or more processors of one or more computing devices, where the one or more processors are operable to execute instructions stored in associated memory, and where the instructions are configured to cause performance of any of the aforementioned methods. Some implementations also include one or more non-transitory computer readable storage media storing computer instructions executable by one or more processors to perform any of the aforementioned methods.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

BRIEF DESCRIPTION OF THE DRAWINGS

In the drawings, like reference characters generally refer to the same parts throughout the different views. Also, the drawings are not necessarily to scale, emphasis instead generally being placed upon illustrating various principles of the embodiments described herein.

Fig. 1 illustrates an example environment in which selected aspects of the present disclosure may be implemented, in accordance with various embodiments.

Fig. 2 illustrates one example counseling session between a subject and a counseling chatbot, in accordance with various embodiments.

Fig. 3A illustrates another example counseling session between a subject and a counseling chatbot, in accordance with various embodiments.

Fig. 3B illustrates another example counseling session between a subject and a counseling chatbot, in accordance with various embodiments.

Fig. 4 depicts an example method for practicing selected aspects of the present disclosure, in accordance with various embodiments.

Fig. 5 depicts an example computing system architecture, in accordance with various embodiments.

Fig. 6 depicts example results that may be achieved using techniques described herein.

DETAILED DESCRIPTION OF EMBODIMENTS

Conventional approaches to help subjects (*e.g.*, patients, clients) change their lifestyle and/or alter their behavior have either been based on health education or health counseling. Passive health education is somewhat effective across a population of subjects, but is less efficient for individual subjects. Scaling of human-provided counselling services across large numbers of subjects, especially in a uniform way, is challenging. In view of the foregoing, various embodiments and implementations of the present disclosure are directed to using artificial intelligence to improve turn-based human-to-computer dialogs. More particularly, but not exclusively, various methods and apparatus disclosed herein relate to applying reinforcement learning to improve turn-based counseling with conversational agents, also referred to herein as "counseling chatbots."

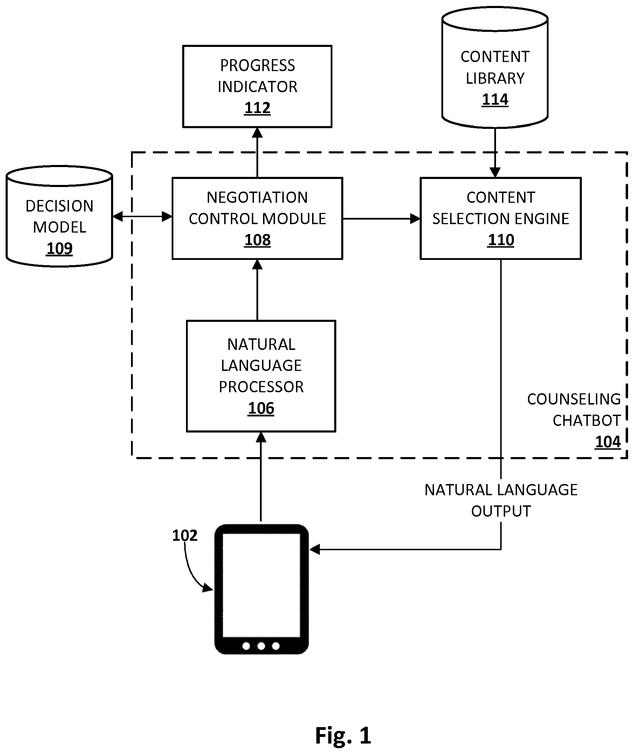

Referring to Fig. 1, an example environment is depicted schematically, showing various components that may be configured to perform selected aspects of the present disclosure. One or more of these components may be implemented using any combination of hardware or software. For example, one or more components may be implemented using one or more microprocessors that execute instructions stored in memory, a field-programmable gate array ("FPGA"), and/or an application-specific integrated circuit ("ASIC"). The connections between the various components represent communication channels that may be implemented using a variety of different networking technologies, such as Wi-Fi, Ethernet, Bluetooth, USB, serial, etc. In embodiments in which the depicted components are implemented as software executed by processor(s), the various components may be implemented across one or more computing systems that may be in communication over one or more networks (not depicted).

A user (not depicted), who may be a "subject" for which a target behavior change is desired (*e.g.*, quit smoking, quit drinking, etc.), may operate one or more client devices 102 to engage in an automated counseling session with a counseling chatbot 104 configured with selected aspects of the present disclosure. The one or more client devices 102 may include, for example, one or more of: a desktop computing device, a laptop computing device, a tablet computing device, a mobile phone computing device, a computing device of a vehicle of the user (*e.g.,* an in-vehicle communications system, an in-vehicle entertainment system, an in-vehicle navigation system), a standalone interactive speaker, a smart appliance such as a smart television, and/or a wearable apparatus of the user that includes a computing device (*e.g.,*

a watch of the user having a computing device, glasses of the user having a computing device, a virtual or augmented reality computing device). Additional and/or alternative client computing devices may be provided.

Counseling chatbot 104 is depicted in dashed lines because while it may appear to the subject as an interactive and logical entity, it is in fact a software-based process that may be implemented using a variety of components. For example, in Fig. 1, counseling chatbot 104 is implemented using a natural language processor 106, a negotiation control module 108, and a content selection engine 110. These components may be implemented using any combination of software and hardware, and may be implemented across one or more computing systems (*e.g.*, sometimes referred to as the "cloud"). In other embodiments, one or more of these components may be omitted and/or combined with other components.

Although not depicted in Fig. 1, in various embodiments, a speech-to-text ("STT") component may be deployed, *e.g.,* on client device 102 and/or elsewhere *(e.g.,* on the "cloud"), that is configured to generate speech recognition output from spoken input provided by the subject. For example, the SST component may employ techniques such as automated speech recognition to generate textual output corresponding to vocal input. Likewise, in various embodiments, a text-to-speech ("TTS") component may be deployed, *e.g.,* on client device 102 and/or elsewhere (*e.g.*, on the "cloud"), that is configured to convert text into computer-generated speech. With components such as STT and TTS, counseling chatbot 104 may be able to communicate with subjects vocally.

Natural language processor 106 may be configured to process natural language input generated by subjects via client devices *(e.g.,* 102) and may generate annotated output for use by one or more other components of counseling chatbot 104. For example, natural language processor 106 may process natural language free-form input that is generated by a subject via one or more user interface input devices of client device 102. The generated annotated output may include one or more annotations of the natural language input and one or more (*e.g.*, all) of the terms of the natural language input.

In some implementations, natural language processor 106 may be configured to identify and annotate various types of grammatical information in natural language input. For example, natural language processor 106 may include a morphological engine (not depicted) that may separate individual words into morphemes and/or annotate the morphemes, *e.g.*, with their classes. Natural language processor 106 may also include a part of speech tagger (not depicted) configured to annotate terms with their grammatical roles. For example, the part of speech tagger may tag each term with its part of speech such as "noun," "verb," "adjective," "pronoun," *etc.* Also, for example, in some embodiments, natural language processor 106 may additionally and/or alternatively include a dependency parser (not depicted) configured to determine syntactic relationships between terms in natural language input. For example, the dependency parser may determine which terms modify other terms, subjects and verbs of sentences, and so forth (*e.g.*, a parse tree) -and may make annotations of such dependencies. In some embodiments, natural language processor 106 may additionally and/or alternatively include a coreference resolver (not depicted) configured to group, or "cluster," references to the same entity based on one or more contextual cues. For example, the coreference resolver may be utilized to resolve the term "it" to "the meeting" in the natural language input "I just got out of a meeting-it really stressed me out so I had a smoke."

In some embodiments, natural language processor 106 may be configured to analyze textual input, which may include speech recognition output generated by the aforementioned STT module and/or typed input from the subject. Based on this analysis, in various embodiments, natural language processor 106 may be configured to determine the subject's state, *e.g.,* by identifying a topic of speech, one or more sentiments, and in some cases classifying the textual input has exhibiting a positive, negative, and/or neutral valance towards a target behavior change. For example, suppose a subject that is trying to quit smoking says something like "I can't take it, I'm stepping out for a smoke." Natural language processor 106 may be configured to analyze this statement and determine that the subject's state includes a negative valence towards the targeted behavior change. In other words, the subject's statement demonstrates movement away from the targeted behavior change of smoking cessation.

In addition to the textual input itself, in some embodiments, natural language processor 106 (or another component depicted in Fig. 1) may rely on other contextual signals to determine a subject's state, *e.g.*, a positive, negative, or neutral valence towards a target behavior change. These contextual signals may be obtained, for instance, from various sensors often integral with mobile phones, smart watches, tablets, in-vehicle computing systems, etc. These sensors may include but are not limited to position coordinate sensors (*e.g.*, GPS, Wi-Fi triangulation), physiological sensors (*e.g.*, heart rate sensors, blood pressure sensors, glucose sensors, breathalyzers, etc.), accelerometers for determining subject activity, and so forth. In some embodiments, natural language processor 106 may annotate the textual input based at least in part on one or more contextual signals. Additionally or alternatively, in various embodiments, one or more contextual signals may be provided to another component, such as negotiation control module 108, for processing.

Negotiation control module 108 may be configured to manage and/or utilize a decision model 109, which as noted above may be used to determine what message y to provide in response to a subject's observed state. In some embodiments, negotiation control module 108 may provide output in the form of a progress indicator 112. As will be described below in more detail, during each turn of a dialog between counseling chatbot 104 and the subject, an instant reward may be calculated. The instant rewards accumulated over a number of turns may, in effect, represent the subject's progress in achieving a target behavior change. In various embodiments, progress indicator 112 may take the form of one or more LEDs (*e.g.,* on a standalone interactive speaker), or various quantitative indicators (*e.g.*, charts, speedometers, progress bars, etc.) that may be rendered on a conventional display (*e.g.*, a touchscreen of a smart phone or smart watch) to convey the subject's overall progress towards the target behavior change.

Decision model 109 may take various forms. In some embodiments, decision model 109 may take the form of a decision matrix, *D*(). In some embodiments, the decision matrix 109 may be updated based on rewards earned by counseling chatbot 104 during automated counseling sessions with the subject, *e.g.*, using an equation such as the following:

$$D(a_k, y_k) = \gamma D(a_k, y_k) + (1 - \gamma) R(A(a_{k-1}, y_k)) \qquad (2)$$

$\gamma$ corresponds to an adaptation coefficient. In some embodiments, $\gamma$ may have a value of 0.9. In some embodiments, decision matrix 109 may be initialized, *e.g.*, by negotiation control module 108, with small random values.

In some embodiments, negotiation control module 108 may be configured to select, based on an action $a_k$ of subject and decision model 109, a "class" or "category" of a message to be provided to the subject by counseling chatbot 104. Various "classes" of messages may be employed, such as informational messages meant to merely inform the subject, stimulation messages meant to elicit a reaction from the subject, and so-called "backchannel" messages meant to cause reflection by the subject.

In some embodiments, a content library 114 may store a corpus of messages and/or message templates, any of which may be used to generate natural language output that can be provided by counseling chatbot 104 to the subject. Each message/message template stored in content library 114 may be associated with a particular class of messages. A message may be as simple as a sequence of words, a phrase, etc., that can be used directly (*e.g.*, verbatim) as natural language output. A message template, by contrast, may include one or more parameters, or "slots," that may be filled with values obtained, for instance, from the subject, from contextual signals associated with the subject, etc. The follow three tables demonstrate non-limiting examples of the types of messages and/or message templates that may be associated with three different classes of messages.

**Table 1: Examples of information messages from counseling chatbot 104**

| |
|---|
| Smoking has halved in the high-educated population in the last 20 years. |
| If you skip the last cigarette in the evening, you will sleep better. |
| If you quit smoking you can easily gain <value> minutes per day more for other things. |
| You have been <value> days without a cigarette. Good progress! |
| Only you can make that decision of quitting. |
| ... |

**Table 2 Examples of stimulating messages from counseling chatbot 104**

| |
|---|
| Did you smoke today? |
| Did it make you feel less stressed? |
| Can you guess how long after you smoked people still smell it? |
| Why do you smoke more in <context A> than in <context B>? |
| Can you take a break without smoking? Just go stand there and breathe. |
| ... |

**Table 3: Examples of backchannel messages from counseling chatbot 104**

| |
|---|
| Well, so you did have one today because <reason> |

(continued)

| Ok, <reason>, right! |
|---|
| Nice progress! |
| Wow! |
| Yeah |
| ... |

In some embodiments, negotiation control module 108 may employ greedy optimization during each turn, such that the goal in each turn is to maximize the immediate reward $R(a_k)$ by selecting a message $y_k$ that maximizes $R(F(a_{k-1}, y_k))$. Suppose the activity emitted by the subject is denoted as $a_k = A(a_{k-1}, y_k)$. In addition, $y_k$ may be considered to represent the indices of the classes of the messages/message templates discussed above, *e.g.*, $y_k = \{0,1,2\}$ and the values of $a_k$ represent at least the following classes of subject utterances with the predefined rewards indicated in the right column:

**Table 4: Classes of Subject Utterances and Associated Rewards**

| $a$ | Action | Reward |
|---|---|---|
| 0 | The utterance contains a negative valence in relation to the target behavior change | 1.4 |
| 1 | The utterance contains a positive valence in relation to the target behavior change | 0.8 |
| 2 | Neutral utterances in relation to the target behavior change | -0.6 |

There are several alternatives for the greedy optimization described above. For example, the goal may instead be to maximize an accumulated reward in two or more consecutive turns, rather than trying to select the reaction corresponding to the maximal anticipated reward in a single turn. For example, in some phase of a counseling session it may be good to allow the subject to continue talking in negative valence without trying to push the subject towards a positive valence, even if the latter would give a higher reward at the level of an individual turn. Additionally or alternatively, in some embodiments, the reward values may depend on a phase of a conversation. For example, in the beginning of a conversation a higher reward may be given for subject actions classified as negative valence than for subject actions classified as positive valence; however, this may be reversed later in the conversation. The weights can also depend on personal preferences of the subject, or some psychological profile of the subject.

In some embodiments, the decision matrix described above by Equation (2) may be updated based on these reward values, *e.g.*, using Equation (2) above. In some such embodiments, negotiation control module 108 may select the class of the message y to be provided by counseling chatbot 104 to the subject by finding a column of a decision matrix that has a maximum accumulated reward, *e.g.*, using an equation such as the following:

$$y = argmax_y[D(a_k, y)] \qquad (3)$$

In some embodiments, decision model 109 used by negotiation control module 108 may take the form of a trained machine learning model, such as a feed forward neural network. In some such embodiments, negotiation control module 108 may retrain decision model 109 at each turn, *e.g.,* by performing back propagation based on the reward value generated based on the subject's reaction (action $a_k$) to adjust one or more weights of one or more hidden layers of the neural network. Thus, a positive reward *(e.g.,* 0.8 from Table 4 above) generated in response to the subject's utterance that contains a positive valence in relation to the target behavior change will reinforce the message class selection made by negotiation control module 108. A negative reward *(e.g.,* 1.4 from Table 4, above) will not reinforce, and may even penalize, the message class selection made by negotiation control module 108.

In some embodiments, content selection engine 110 may receive, from negotiation control module 108, the class of message y that is to be provided by counseling chatbot 104 to the subject. Based on this received class, and in some cases on one or more additional (*e.g.*, contextual) signals, content selection engine 110 may select an actual message and/or message template from content library 114 that is associated with the received class. These one or more additional signals may include, for instance, contextual signals (described previously), demographic data points about the subject (*e.g.*, age, gender, etc.), and so forth. For example, one message that conveys the effects of smoking on erectile dysfunction may be more suitable for male subjects, and another message that conveys the effects of smoking on menopause may be more suitable for female subjects.

In some embodiments, content selection engine 110 may be configured to complete a message template, *e.g.*, by filling the parameters or slots with values. These values may be, for instance, solicited from the subject by counseling chatbot 104, or determined based on contextual signals received, for instance, from one or more computing devices *(e.g.,* 102) operated by the subject. When a message template from a "reflection" class is selected, oftentimes such a message template may be filled, *e.g.*, by content selection engine 110, using the subject's own words, or at least variations thereof.

The following is an example that demonstrates operation of the various components of Fig. 1. Assume counseling chatbot 104 is being employed to influence a subject to quit smoking. As an attempt to determine whether the subject is exhibiting positive or negative valence towards this target behavior change, counseling chatbot 104, and in particular, negotiation control module 108, may select, as a class of message to be output to the subject, the class "stimulating messages." Based on this class, content selection engine 110 may select, from content library 114, the message, "When did you last smoke a cigarette?" Once this message is output by counseling chatbot 104, suppose the subject responds (as an action *a*), "I had a cigarette this morning because I'm super stressed." Natural language processor 106 may identify, from text generated from this utterance, a negative valence towards the target behavior change. Natural language processor 106 may provide data indicative of this negative valence (*e.g.*, annotated version of the subject's textual input) to negotiation control module 108. Based on the subject's perceived state, as represented by the subject's utterance (action *a*), negotiation control module 108 may select a class of the next message to be delivered by counseling chatbot 104 to subject, *e.g.*, "backchannel." Based on this selected class, content selection engine 110 may select, from content library 114 for output to the subject, a message such as "OK, you're stressed, right!"

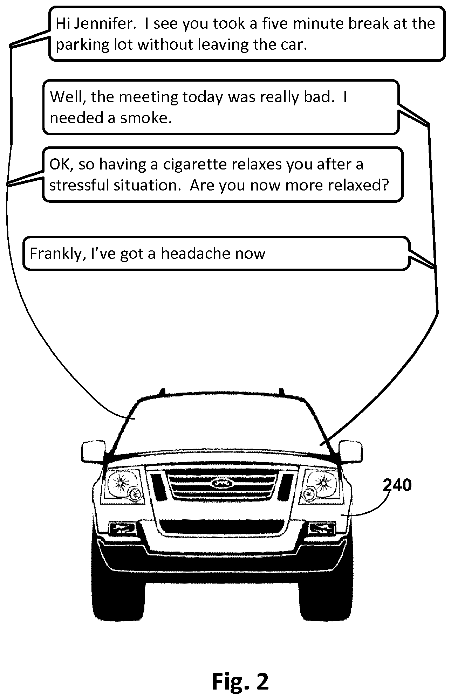

Fig. 2 depicts a scenario in which a subject ("Jennifer") engages with counseling chatbot 104 using an in-vehicle computing system of a vehicle 240. In this example, counseling chatbot 104 determines, *e.g.*, based on one or more contextual signals generated by Jennifer's smart phone, that Jennifer sat in vehicle for five minutes. This detected activity by Jennifer may be interpreted, *e.g.*, by natural language processor 106 and/or by negotiation control module 108, as a neutral valence towards a target behavior change of Jennifer to quit smoking. Thus, negotiation control module 108 may select a message class of "stimulating" in order to solicit an utterance from Jennifer. Based on the selected message class, "stimulating," content selection engine 110 may select, from content library 114, a message template associated with the "stimulating" message class. This message template may include slots, *e.g.,* "Hi <subject>. I see you took a <time interval> break at the <location> without leaving the car." Content selection engine 110 may fill these slots with values obtained, for instance, from contextual signals associated with Jennifer, such that counseling chatbot 104 ultimately provides the natural language output, "Hi Jennifer. I see you took a five minute break at the parking lot without leaving the car."

In response, Jennifer explains, "Well, the meeting today was really bad. I needed a smoke." Jennifer's utterance may be speech recognized to generate textual input, and the textual input may be analyzed/annotated by natural language processor 106 as described above, *e.g.,* to include annotations related to sentiment, valence towards a target behavior change, etc. Negotiation control module 108 may select "backchannel" as a class from which the next message should be selected for provision to Jennifer. Content selection engine 110 may use the "backchannel" class, as well as one or more additional signals (*e.g.*, contextual signals), to select the backchannel class message, "OK, so having a cigarette relaxes you after a stressful situation." This message is meant to cause Jennifer to reflect on her decision.

In some embodiments, negotiation control module 108 may provide multiple classes to content selection engine 110, so that content selection engine 110 can provide multiple different types of messages in a single turn. An example of this is seen in Fig. 2, where negotiation control module 108 also provided "stimulating" as a message class to content selection engine 110. Consequently, immediately after providing the message, "OK, so having a cigarette relaxes you after a stressful situation," counseling chatbot 104 then asks, "Are you now more relaxed?" Jennifer's next statement, "Frankly, I've got a headache now.", could be interpreted as either a positive valence towards the target behavior change (i.e. it demonstrates that Jennifer regrets that she smoked) or at least a neutral valance towards the target behavior change. In the former case, counseling chatbot 104 may be given a positive reward to reinforce the decision to select one or both statements, "OK, so having a cigarette relaxes you after a stressful situation," and "Are you now more relaxed?"

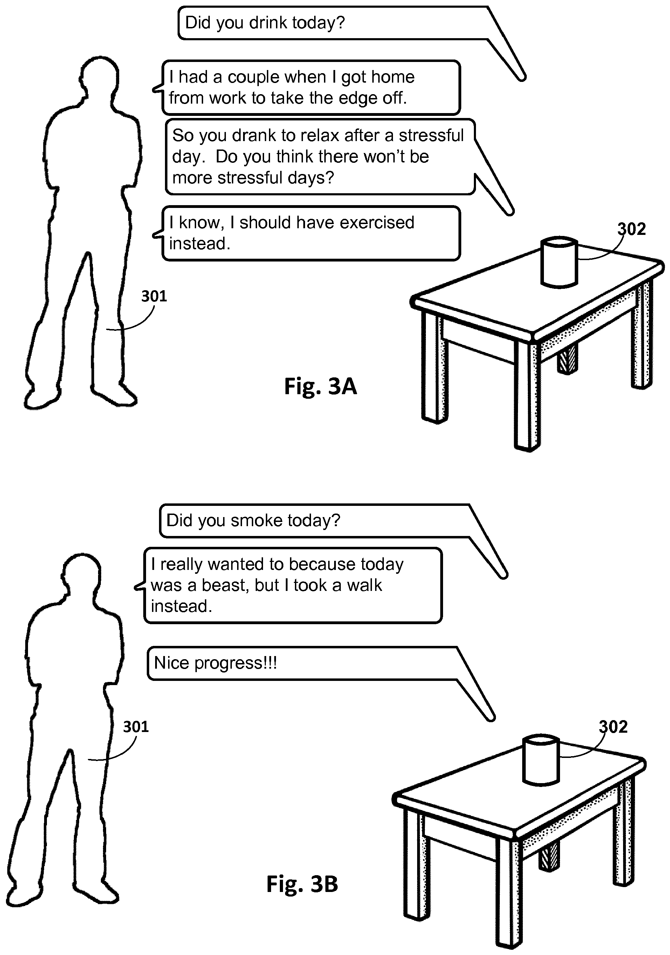

In Figs. 3A and 3B, a subject 301 interacts with counseling chatbot 104 via a client device 302 taking the form of a standalone interactive speaker. In Fig. 3A, the target behavior change of subject 301 is to quit drinking alcohol. Counseling chatbot 104 begins by asking subject 301, "Did you drink today?" This message takes the form of a solicitation that is selected by content selection engine 110 based on a "stimulating" message class selected by negotiation control module 108, *e.g.,* to stimulate an action from subject 301 that can be used to further the counseling. And in fact, subject 301 responds, "I had a couple when I got home from work to take the edge off."

This utterance by subject 301 may be interpreted, *e.g.*, by natural language processor 106 and/or negotiation control module 108, as a negative valence towards the target behavior or abstention from alcohol. Consequently, negotiation control module 108 may select, *e.g.,* based on decision model 109, the "backchannel" message class, which may result in counseling chatbot 104 making a reflective statement, "So you drank to relax after a stressful day." Additionally or alternatively, negotiation control module 108 may select, *e.g.,* based on decision model 109, the "stimulating" message class, which may result in counseling chatbot 104 asking, "Do you think there won't be more stressful days?" The user responds with an utterance-"I know. I should have exercised instead"-that contains a positive valence towards the target behavior change; i.e. an acknowledgement that having the drink was undesirable and that a healthier alternative (exercise) would have been preferable. Consequently, a positive reward may be generated and used to reinforce the decision of counseling chatbot 104 to ask, "Do you think there won't be more stressful days?"

In Fig. 3B, subject 301 is now trying to quit smoking. Counseling chatbot 104 asks, "Did you smoke today?" Subject 301 responds, "I really wanted to because today was a beast, but I took a walk instead." This utterance clearly contains a positive valence towards a target behavior change of smoking cessation. Consequently, counseling chatbot 104 may output an encouraging message, "Nice progress!!!"

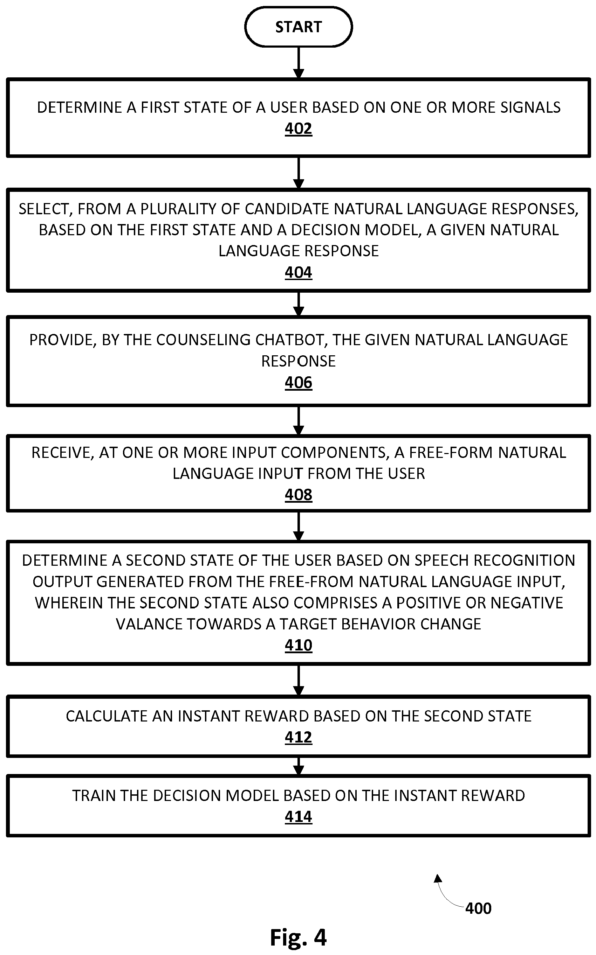

Fig. 4 depicts an example method 400 for practicing selected aspects of the present disclosure, in accordance with various embodiments. For convenience, the operations of the flow chart are described with reference to a system that performs the operations. This system may include various components of various computer systems, including the components of counseling chatbot 104 depicted in Fig. 1. Moreover, while operations of method 400 are shown in a particular order, this is not meant to be limiting. One or more operations may be reordered, omitted or added.

At block 402, the system may determine a first state of a subject based on one or more signals. As described previously, the first state may be determined based on various signals, such as contextual signals (*e.g.*, GPS, physiological sensors, etc.), textual input from the subject (*e.g.,* typed or converted from speech), and so forth. In some embodiments, the first state may include a positive or negative valence towards a target behavior change, although this is not necessarily required. A message can be selected based on the subject's state even if the subject's state does not include a valence towards the target behavior change.

At block 404, the system may select, from a plurality of candidate natural language responses (*e.g.*, messages in content library 114), based on the first state and a decision model (*e.g.,* 109), a given natural language response. For example, as explained above, negotiation control module 108 may select a message class of the next message to be delivered by counseling chatbot 104 based on the decision model and the first state. If the decision model is a neural network, a vector corresponding to the first state may be applied as input across the neural network to generate output indicative, for instance, of a plurality of probabilities associated with a plurality of candidate message classes. In some embodiments, the highest probability message class may be selected always, or a message class may be selected stochastically based on the probabilities.

At block 406, the system, *e.g.*, by way of counseling chatbot 104, may provide, at one or more output components of one or more computing devices (*e.g.,* 102) operated by the subject to engage in the human-to-computer dialog with counseling chatbot 104, the given natural language response. At block 408, the system may receive, *e.g.,* at one or more input components of one or more of the computing devices (102), a free-form natural language input from the subject.

At block 410, the system may determine a second state of the subject based on speech recognition output generated from the free-from natural language input. (If the free-form natural language input was typed by the subject, rather than uttered, then speech recognition may not be necessary). In various embodiments, the second state may include a positive or negative (or neutral) valance towards the target behavior change. This valence may be detected, *e.g.,* by natural language processor 106 and/or by negotiation control module 108.

Based on the second state, at block 412, the system may calculate an instant reward based on the second state. At block 414, the system may train the decision model based on the instant reward. For example, if the decision model is a decision matrix, then equations such as Equations (1) and/or (2) set forth previously may be used to update the decision matrix. If the decision model is a neural network, then the instant reward may be used to perform techniques such as gradient descent and/or back propagation to train the model.

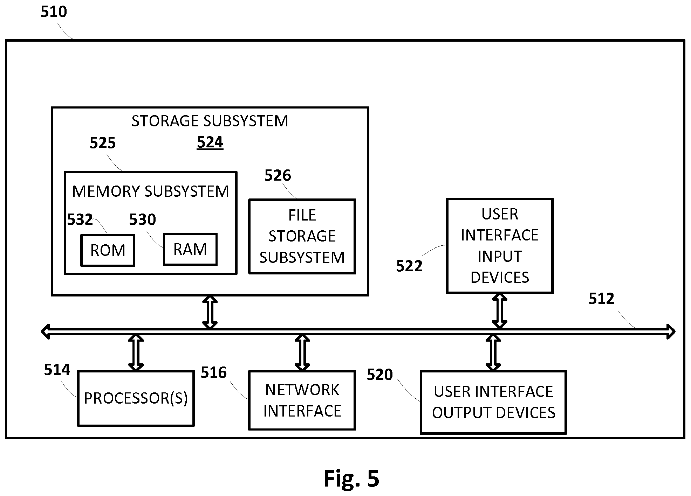

Fig. 5 is a block diagram of an example computing device 510 that may optionally be utilized to perform one or more aspects of techniques described herein. In some implementations, one or more of a client computing device, user-controlled resources engine 130, and/or other component(s) may comprise one or more components of the example computing device 510.

Computing device 510 typically includes at least one processor 514 which communicates with a number of peripheral devices via bus subsystem 512. These peripheral devices may include a storage subsystem 524, including, for example, a memory subsystem 525 and a file storage subsystem 526, user interface output devices 520, user interface input devices 522, and a network interface subsystem 516. The input and output devices allow user interaction with computing device 510. Network interface subsystem 516 provides an interface to outside networks and is coupled to corresponding interface devices in other computing devices.

User interface input devices 522 may include a keyboard, pointing devices such as a mouse, trackball, touchpad, or graphics tablet, a scanner, a touchscreen incorporated into the display, audio input devices such as voice recognition systems, microphones, and/or other types of input devices. In general, use of the term "input device" is intended to include all possible types of devices and ways to input information into computing device 510 or onto a communication network.

User interface output devices 520 may include a display subsystem, a printer, a fax machine, or non-visual displays such as audio output devices. The display subsystem may include a cathode ray tube (CRT), a flat-panel device such as a liquid crystal display (LCD), a projection device, or some other mechanism for creating a visible image. The display subsystem may also provide non-visual display such as via audio output devices. In general, use of the term "output device" is intended to include all possible types of devices and ways to output information from computing device 510 to the user or to another machine or computing device.

Storage subsystem 524 stores programming and data constructs that provide the functionality of some or all of the modules described herein. For example, the storage subsystem 524 may include the logic to perform selected aspects of the method of Fig. 4, as well as to implement various components depicted in Fig. 1.

These software modules are generally executed by processor 514 alone or in combination with other processors. Memory 525 used in the storage subsystem 524 can include a number of memories including a main random access memory (RAM) 530 for storage of instructions and data during program execution and a read only memory (ROM) 532 in which fixed instructions are stored. A file storage subsystem 526 can provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem 526 in the storage subsystem 524, or in other machines accessible by the processor(s) 514.

Bus subsystem 512 provides a mechanism for letting the various components and subsystems of computing device 510 communicate with each other as intended. Although bus subsystem 512 is shown schematically as a single bus, alternative implementations of the bus subsystem may use multiple busses.

Computing device 510 can be of varying types including a workstation, server, computing cluster, blade server, server farm, or any other data processing system or computing device. Due to the ever-changing nature of computers and networks, the description of computing device 510 depicted in Fig. 5 is intended only as a specific example for purposes of illustrating some implementations. Many other configurations of computing device 510 are possible having more or fewer components than the computing device depicted in Fig. 5.

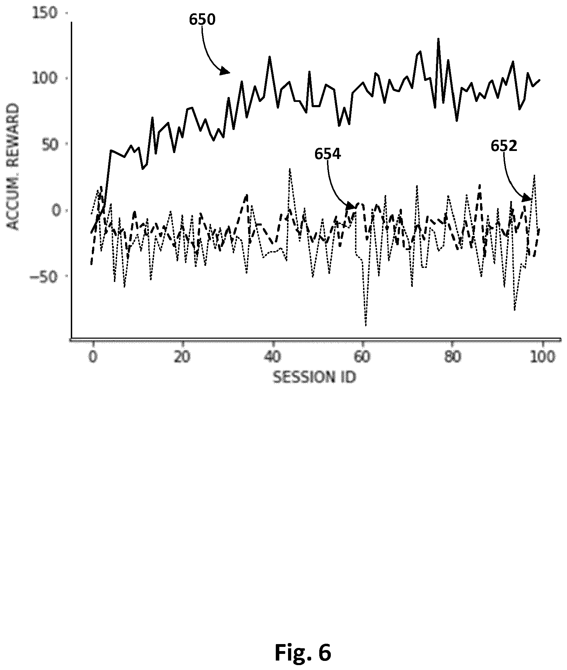

Fig. 6 depicts example results that may be obtained using techniques described herein. In Fig. 6, the first data 650 represents accumulated rewards (y axis) achieved by a counseling chatbot across multiple sessions (x axis), wherein the chatbot uses a decision model trained using the reinforcement learning techniques described herein. The second data 652 represents accumulated rewards achieved by a counseling chatbot that selects output messages randomly, across multiple sessions. The third data 654 represents accumulated rewards achieved by a counseling chatbot simulated based on a Markov model derived from a real counseling session. These results demonstrate that the counseling chatbot that relies on a decision model trained using reinforcement learning techniques described herein performs significantly better (i.e., obtains a much higher cumulative reward), especially after a few turns. As noted previously, the accumulated reward represented by first data 650 may in some embodiments be output, *e.g.*, using LEDs of a standalone interactive speaker, or a conventional touchscreen, to apprise a subject of his or her overall progress in achieving a target behavior change.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

**Claims**

1.  A method implemented by one or more processors as part of a human-to-computer dialog between a subject and a counseling chatbot (104), the method comprising:

    determining (402) a first state of the subject based on one or more signals;
    selecting (404), from a plurality of candidate natural language responses, based on the first state and a decision model (109), a given natural language response;
    providing (406), by the counseling chatbot, at one or more output components of one or more computing devices operated by the subject to engage in the human-to-computer dialog with the counseling chatbot, the given natural language response;
    receiving (408), at one or more input components of one or more of the computing devices, a free-form natural language input from the subject;
    determining (410) a second state of the subject based on speech recognition output generated from the free-from natural language input, wherein the second state comprises a positive or negative valance towards a target behavior change;
    calculating (412) an instant reward based on the second state; and
    training (414) the decision model based on the instant reward.

2. The method of claim 1, wherein the decision model comprises a decision matrix, and training the decision model comprises updating the decision matrix based on the instant reward.

3. The method of claim 1, wherein the decision model comprises a neural network.

4. The method of claim 3, wherein training the neural network comprises applying back propagation to adjust one or more weights associated with one or more hidden layers of the neural network, wherein applying the back propagation is based on the instant reward.

5. The method of claim 1, wherein the one or more signals comprise speech recognition generated from a first free-form natural language input, and the free-form natural language input comprises a second free-form natural language input.

6. The method of claim 1, further comprising determining, based at least in part on the instant reward and other instance rewards calculated during the human-to-computer dialog, a cumulative reward.

7. The method of claim 6, further comprising providing, at one or more visual output components of one or more of the computing devices operated by the subject, a visual indication of the cumulative reward.

8. The method of claim 1, wherein training the decision model includes maximizing a cumulative mean reward $R_C$ given by the following equation:

$$R_c = \frac{1}{K} \sum_{k}^{K} R(a_k)$$

wherein K is a positive integer corresponding to a number of turns in the human-to-computer dialog, and $a_k$ represents an action at a given turn k, and $R(a_k)$ represents an instant reward at a given turn k.

9. The method of claim 1, wherein the plurality of candidate natural language responses include: a first set of informational candidate responses; a second set of candidate responses designed to stimulate a response from the subject; and a third set of candidate responses designed to simulate listening or reflection on part of the counseling chatbot.

10. A system comprising one or more processors and memory operably coupled with the one or more processors, wherein the memory stores instructions that, in response to execution of the instructions by one or more processors, cause the one or more processors to perform the following operations as part of a human-to-computer dialog between a subject and a counseling chatbot (104):

   determining (402) a first state of the subject based on one or more signals;
   selecting (404), from a plurality of candidate natural language responses, based on the first state and a decision model (109), a given natural language response;
   providing (406), by the counseling chatbot, at one or more output components of one or more computing devices operated by the subject to engage in the human-to-computer dialog with the counseling chatbot, the given natural language response;
   receiving (408), at one or more input components of one or more of the computing devices, a free-form natural language input from the subject;
   determining (410) a second state of the subject based on speech recognition output generated from the free-from natural language input, wherein the second state comprises a positive or negative valance towards a target behavior change;
   calculating (412) an instant reward based on the second state; and
   training (414) the decision model based on the instant reward.

11. At least one non-transitory computer-readable medium comprising instructions that, in response to execution of the instructions by one or more processors, cause the one or more processors to perform the following operations as part of a human-to-computer dialog between a subject and a counseling chatbot:

   determining (402) a first state of the subject based on one or more signals;

selecting (404), from a plurality of candidate natural language responses, based on the first state and a decision model (109), a given natural language response;

providing (406), by the counseling chatbot, at one or more output components of one or more computing devices operated by the subject to engage in the human-to-computer dialog with the counseling chatbot, the given natural language response;

receiving (408), at one or more input components of one or more of the computing devices, a free-form natural language input from the subject;

determining (410) a second state of the subject based on speech recognition output generated from the free-from natural language input, wherein the second state comprises a positive or negative valance towards a target behavior change;

calculating (412) an instant reward based on the second state; and

training (414) the decision model based on the instant reward.

12. The at least one non-transitory computer-readable medium of claim 11, wherein the decision model comprises a decision matrix, and training the decision model comprises updating the decision matrix based on the instant reward.

**Fig. 1**

Hi Jennifer.  I see you took a five minute break at the parking lot without leaving the car.

Well, the meeting today was really bad.  I needed a smoke.

OK, so having a cigarette relaxes you after a stressful situation.  Are you now more relaxed?

Frankly, I've got a headache now

240

**Fig. 2**

Did you drink today?

I had a couple when I got home from work to take the edge off.

So you drank to relax after a stressful day. Do you think there won't be more stressful days?

I know, I should have exercised instead.

301

302

**Fig. 3A**

Did you smoke today?

I really wanted to because today was a beast, but I took a walk instead.

Nice progress!!!

301

302

**Fig. 3B**

START

DETERMINE A FIRST STATE OF A USER BASED ON ONE OR MORE SIGNALS
**402**

SELECT, FROM A PLURALITY OF CANDIDATE NATURAL LANGUAGE RESPONSES, BASED ON THE FIRST STATE AND A DECISION MODEL, A GIVEN NATURAL LANGUAGE RESPONSE
**404**

PROVIDE, BY THE COUNSELING CHATBOT, THE GIVEN NATURAL LANGUAGE RESPONSE
**406**

RECEIVE, AT ONE OR MORE INPUT COMPONENTS, A FREE-FORM NATURAL LANGUAGE INPUT FROM THE USER
**408**

DETERMINE A SECOND STATE OF THE USER BASED ON SPEECH RECOGNITION OUTPUT GENERATED FROM THE FREE-FROM NATURAL LANGUAGE INPUT, WHEREIN THE SECOND STATE ALSO COMPRISES A POSITIVE OR NEGATIVE VALANCE TOWARDS A TARGET BEHAVIOR CHANGE
**410**

CALCULATE AN INSTANT REWARD BASED ON THE SECOND STATE
**412**

TRAIN THE DECISION MODEL BASED ON THE INSTANT REWARD
**414**

400

## Fig. 4

**510**

STORAGE SUBSYSTEM

**525**          **524**

**526**

MEMORY SUBSYSTEM

**532**      **530**

ROM        RAM

FILE
STORAGE
SUBSYSTEM

USER
INTERFACE
INPUT
DEVICES

**522**

**512**

**514**   PROCESSOR(S)

**516**   NETWORK
INTERFACE

**520**   USER INTERFACE
OUTPUT DEVICES

**Fig. 5**

**Fig. 6**

Europäisches
Patentamt

European
Patent Office

Office européen
des brevets

**EUROPEAN SEARCH REPORT**

Application Number

EP 18 16 3225

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | IULIAN V SERBAN ET AL: "A Deep Reinforcement Learning Chatbot", CORR (ARXIV), vol. 1709.02349v2, 5 November 2017 (2017-11-05), pages 1-40, XP055507018, * paragraph [0001] - paragraph [03.1] * * paragraph [0004] - paragraph [04.8] * * paragraph [07.1] - paragraph [07.1] * ----- | 1-12 | INV. G06N3/00 G06N5/04 |
| A | KATHLEEN KARA FITZPATRICK ET AL: "Delivering Cognitive Behavior Therapy to Young Adults With Symptoms of Depression and Anxiety Using a Fully Automated Conversational Agent (Woebot): A Randomized Controlled Trial", JMIR MENTAL HEALTH, vol. 4, no. 2, 6 June 2017 (2017-06-06), page e19, XP055506968, DOI: 10.2196/mental.7785 * abstract; figure 3 * * page 2 - page 3 * * paragraph [Discussion] * ----- | 1-12 | |
| A | ADAM MINER ET AL: "Conversational Agents and Mental Health : Theory-Informed Assessment of Language and Affect", PROCEEDINGS OF THE FOURTH INTERNATIONAL CONFERENCE ON HUMAN AGENT INTERACTION, HAI '16, 4 October 2016 (2016-10-04), pages 123-130, XP055506995, New York, New York, USA DOI: 10.1145/2974804.2974820 ISBN: 978-1-4503-4508-8 * the whole document * ----- | 1-12 | |

TECHNICAL FIELDS
SEARCHED (IPC)

G06N

The present search report has been drawn up for all claims

| Place of search | Date of completion of the search | Examiner |
|---|---|---|
| The Hague | 17 September 2018 | Cilia, Elisa |

EPO FORM 1503 03.82 (P04C01)